# FASCICULE DE BREVET EUROPEEN

(11) **EP 3 773 161 B1**
(45) Date de publication et mention de la délivrance du brevet: **16.07.2025**
(21) Numéro de dépôt: 19718802.2
(22) Date de dépôt: 26.03.2019
(51) Int. Cl.: A61B 5/00, A61B 5/361, G16H 50/20, G16H 50/30, G16H 50/70

(54) **DISPOSITIF INFORMATIQUE DE DÉTECTION DE TROUBLES DU RYTHME CARDIAQUE**
RECHNERVORRICHTUNG ZUR ERFASSUNG VON HERZRHYTHMUSSTÖRUNGEN
COMPUTING DEVICE FOR DETECTING HEART RHYTHM DISORDERS

(30) Priorité: 30.03.2018 FR 1852850
(43) Date de publication de la demande: 17.02.2021
(73) Titulaire: SUBSTRATE HD, 13008 Marseille (FR)
(72) Inventeur: MOHR DURDEZ, Théophile, 13008 Marseille (FR)
(74) Mandataire: Cabinet Netter
(86) Numéro de dépôt international: PCT/FR2019/050682
(87) Numéro de publication internationale: WO 2019/186050

(56) Documents cités:
- US-A1- 2011 098 775
- US-A1- 2011 270 346
- US-A1- 2016 022 162
- US-A1- 2017 032 221
- US-A1- 2017 032 221
- SANJIV M. NARAYAN ET AL: "Evaluating Fluctuations in Human Atrial Fibrillatory Cycle Length Using Monophasic Action Potentials", PACE - PACING AND CLINICAL ELECTROPHYSIOLOGY., vol. 29, no. 11, 7 November 2006 (2006-11-07), US, pages 1209 - 1218, XP055416706, ISSN: 0147-8389, DOI: 10.1111/j.1540-8159.2006.00525.x

## Description

L'invention concerne le domaine des dispositifs informatiques de détection de troubles du rythme cardiaque, et en particulier la détection de zones cardiaques favorisant la fibrillation atriale.

Le domaine de la détection de zones cardiaques favorisant la fibrillation atriale comprend des dispositifs informatiques fonctionnant en temps différé mettant en œuvre des logiciels comme les logiciels Topera et CardioInsight, ainsi que des dispositifs informatiques fonctionnant en temps réels mettant en œuvre des logiciels comme le logiciel CARTO.

Le logiciel Topera a pour objectif de reconstruire l'activation électrique des oreillettes du cœur. L'acquisition des signaux est réalisée au moyen d'un « basket catheter » (cathéter qui se déploie sur toute l'oreillette). L'analyse se fait en temps différé, plus de 2 minutes après le début de l'acquisition. Cette solution est gênante car ce type de cathéter est difficile à mettre en place et car le contact des électrodes n'est pas assuré. Le temps d'analyse est très long, et la reconstruction ne permet pas d'avoir des cartes haute définition, voire échoue dans le cas d'activation électriques complexes (qui représentent 70% des cas de fibrillation atriale) du fait d'une reconstruction trop simpliste.

Le logiciel CardioInsight a pour objectif de reconstruire l'activité électrique du cœur à l'aide d'un gilet de mesure d'électrocardiogramme multi-électrodes sur la peau du patient. Le patient porte le gilet avant l'opération, les données sont analysées puis accessibles pendant l'opération. L'analyse se fait en temps différé (plus de 15 minutes) après le début de l'acquisition. Cette solution présente le désavantage d'un temps de calcul très important, qui impose un temps différé la rendant difficile à mettre en place (en effet, le patient doit venir quelques jours avant et porter le gilet pour que les données puissent être extraites avant opération), et un coût très important. En outre, la reconstruction ne permet pas d'avoir des cartes haute définition du fait des mesures trop périphériques et distantes, et échoue dans le cas d'activation électriques complexes du fait d'une reconstruction trop simpliste.

Le logiciel CARTO édité par la société Biosense & Webster permet de mettre en œuvre des algorithmes afin de détecter des arythmies cardiaques.

Ainsi l'algorithme de CFAE (Complex Fractionated Atrial Electrograms) calcule le nombre de points d'inflexion (variation du signe de la dérivée) dans les signaux et produit des cartes de couleur en temps réel. Le praticien interprète alors ces cartes complexes pour déterminer les lieux d'intérêt. Cet algorithme est peu spécifique et relativement simple.

L'algorithme Ripple (Ripple Mapping) est un module du logiciel CARTO qui permet de reproduire l'activité électrique des oreillettes après un premier passage d'un cathéter. L'amplitude comme la propagation des ondes électriques sont rendues visibles au travers de ce module. Il en résulte des cartes extrêmement complexes à comprendre pour le praticien. Si cela peut fonctionner dans les cas simples, l'analyse échoue dans le cas d'activation électriques complexes car les cartes sont trop difficiles à interpréter.

Le document US 2016/022162 A1 divulgue un dispositif de détermination d'un risque cardiaque sur la base de données d'électrogrammes cardiaques ; ce dispositif met en œuvre une pluralité de modèles de classification de détection de troubles du rythme cardiaque.

Il apparaît donc qu'aucun dispositif informatique de détection de troubles du rythme cardiaque n'est disponible qui permette à un praticien de détecter de manière rapide et fiable, y compris dans le cas d'activations électriques complexes, des troubles du rythme cardiaque.

L'invention vient améliorer la situation. À cet effet, l'invention propose un dispositif de détection de zones cardiaques favorisant la fibrillation atriale qui comprend une mémoire agencée pour recevoir des données d'électrogrammes cardiaques et stockant des données définissant un premier modèle de classification de détection de troubles du rythme cardiaque et un second modèle de classification de détection de troubles du rythme cardiaque, le premier modèle de classification de détection de troubles du rythme cardiaque découlant du traitement par apprentissage automatique de données associées à des électrogrammes cardiaques ayant une première durée, et le deuxième modèle de classification de détection de troubles du rythme cardiaque découlant du traitement par apprentissage automatique de données associées à des électrogrammes cardiaques ayant une deuxième durée, la première durée étant inférieure à la deuxième durée, un classifieur agencé pour analyser des données d'électrogrammes cardiaques sur la base d'un modèle de classification, et pour renvoyer une valeur de classification, et un pilote agencé pour stocker dans la mémoire des données d'électrogrammes cardiaques reçues en entrée, d'une part pour regrouper ces données selon la première durée et les analyser avec le classifieur sur la base du premier modèle de classification de détection de troubles du rythme cardiaque et d'autre part pour regrouper ces données selon la deuxième durée et les analyser avec le classifieur sur la base du deuxième modèle de classification de détection de troubles du rythme cardiaque, et pour retourner des données d'alerte lorsque l'analyse par le classifieur sur la base du premier modèle de classification retourne une valeur de classification associée à un trouble du rythme cardiaque.

Ce dispositif est particulièrement avantageux car il permet à une détection beaucoup plus fiable et rapide que les dispositifs existants.

Dans diverses variantes, le dispositif selon l'invention peut présenter une ou plusieurs des caractéristiques suivantes :
- la deuxième durée est un multiple entier de la première durée, et dans lequel le pilote est agencé pour appliquer le deuxième modèle de classification de détection de troubles du rythme cardiaque de sorte que la valeur de classification pour des données d'électrogrammes ayant la deuxième durée correspond à une combinaison linéaire des valeurs de classification obtenues en découpant les données d'électrogrammes ayant la deuxième durée en une pluralité de sous-groupes de données d'électrogrammes ayant la première durée et en appliquant le premier modèle de classification de détection de troubles du rythme cardiaque à chacun de ces sous-groupes de données d'électrogrammes,
- le pilote est agencé pour calculer une moyenne pondérée des valeurs de classification obtenues en appliquant le premier modèle de classification de détection de troubles du rythme cardiaque à chacun des sous-groupes de données d'électrogrammes,
- le dispositif selon l'une des revendications précédentes, comprenant en outre un moteur d'apprentissage automatique agencé pour déterminer le premier modèle de classification de détection de troubles du rythme cardiaque et le deuxième modèle de classification de détection de troubles du rythme cardiaque, et
- le moteur d'apprentissage est agencé pour exécuter un apprentissage choisi parmi un apprentissage supervisé, un apprentissage semi-supervisé, un apprentissage non-supervisé, ou une combinaison de deux ou plus de ces apprentissages.

Cette divulgation concerne également un procédé de détection de troubles du rythme cardiaque mise en œuvre par ordinateur comprenant les opérations suivantes :
a) recevoir des données d'électrogrammes cardiaques,
b) regrouper des données reçues à l'opération a) d'une part selon une première durée et d'autre part selon une deuxième durée, la première durée étant inférieure à la deuxième durée,
c) analyser les données regroupées selon la première durée sur la base d'un premier modèle de classification de détection de troubles du rythme cardiaque, le premier modèle de classification de détection de troubles du rythme cardiaque découlant du traitement par apprentissage automatique de données associées à des électrogrammes cardiaques ayant la première durée, et déterminer une première valeur de classification,
d) analyser les données regroupées selon la deuxième durée sur la base d'un deuxième modèle de classification de détection de troubles du rythme cardiaque, le deuxième modèle de classification de détection de troubles du rythme cardiaque découlant du traitement par apprentissage automatique de données associées à des électrogrammes cardiaques ayant la deuxième durée, et déterminer une deuxième valeur de classification, et
e) émettre des données d'alerte lorsque la première valeur de classification est associée à un trouble du rythme cardiaque.

En tant que tel, un tel procédé ne fait pas partie de l'invention revendiquée.

Dans diverses variantes, le dispositif selon l'invention peut présenter une ou plusieurs des caractéristiques suivantes :
- la deuxième durée est un multiple entier de la première durée, et le calcul de la deuxième valeur de classification de l'opération d) comprend le calcul d'une combinaison linéaire de premières valeurs de classification obtenues en découpant les données d'électrogrammes ayant la deuxième durée en une pluralité de sous-groupes de données d'électrogrammes ayant la première durée et en appliquant l'opération c) à ces sous-groupes de données d'électrogrammes,
- la combinaison linéaire de l'opération d) comprend le calcul d'une moyenne pondérée des premières valeurs de classification obtenues en appliquant le premier modèle de classification de détection de troubles du rythme cardiaque aux sous-groupes de données d'électrogrammes ayant la première durée,
- le premier modèle de classification de détection de troubles du rythme cardiaque et le deuxième modèle de classification de détection de troubles du rythme cardiaque sont obtenus par apprentissage automatique, et
- l'apprentissage automatique est un apprentissage choisi parmi un apprentissage supervisé, un apprentissage semi-supervisé, un apprentissage non-supervisé, ou une combinaison de deux ou plus de ces apprentissages.

D'autres caractéristiques et avantages de l'invention apparaîtront mieux à la lecture de la description qui suit, tirée d'exemples donnés à titre illustratif et non limitatif, tirés des dessins sur lesquels :
- la Figure 1 représente un mode de réalisation d'un dispositif selon l'invention, et
- la Figure 2 représente un exemple de mise en œuvre d'une fonction par le dispositif de la Figure 1.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

La présente description est de nature à faire intervenir des éléments susceptibles de protection par le droit d'auteur et/ou le copyright. Le titulaire des droits n'a pas d'objection à la reproduction à l'identique par quiconque du présent document de brevet ou de sa description, telle qu'elle apparaît dans les dossiers officiels. Pour le reste, il réserve intégralement ses droits.

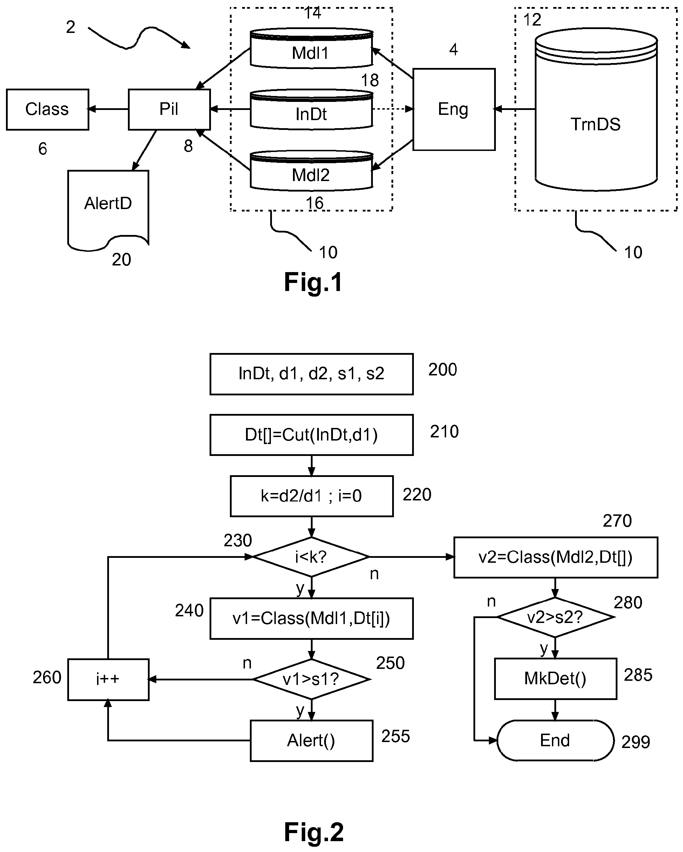

La Figure 1 représente un mode de réalisation d'un dispositif de détection de troubles du rythme cardiaque selon l'invention.

Le dispositif 2 comprend un moteur 4, un classifieur 6 et un pilote 8 une mémoire 10.

Dans l'exemple décrit ici, le dispositif 2 est mis en œuvre sur un ordinateur qui reçoit des données d'électrogrammes cardiaque en entrée et offre un retour à un praticien via une interface graphique non représentée. Cet ordinateur est ici de type PC muni du système d'exploitation Windows 10, d'une carte graphique capable de gérer un ou plusieurs affichages. Bien sûr, il pourra être réalisé de manière différente, avec un système d'exploitation différent, avec une communication avec ou sans fil avec le ou les affichages. Le terme ordinateur doit être interprété au sens large du terme. Par exemple, il pourrait être une tablette ou un smartphone, un terminal d'interaction avec un serveur de calcul, ou un élément sur une grille de ressources distribuées, etc.

Le moteur 4, le classifieur 6 et le pilote 8 sont ici des programmes exécutés par le processeur de l'ordinateur. En variante, un ou plusieurs de ces éléments pourrait être mis en œuvre de manière différente au moyen d'un processeur dédié. Par processeur, il doit être compris tout processeur adapté aux traitements de données décrits plus bas. Un tel processeur peut être réalisé de toute manière connue, sous la forme d'un microprocesseur pour ordinateur personnel, d'une puce dédiée de type FPGA ou SoC (« system on chip » en anglais), d'une ressource de calcul sur une grille, d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeur et de circuits électroniques peut également être envisagée.

La mémoire 10 peut être tout type de stockage de données propre à recevoir des données numériques : disque dur, disque dur à mémoire flash (SSD en anglais), mémoire flash sous toute forme, mémoire vive, disque magnétique, stockage distribué localement ou dans le cloud, etc. Les données calculées par le dispositif 2 peuvent être stockées sur tout type de mémoire similaire à la mémoire 10, ou sur celle-ci. Ces données peuvent être effacées après que le dispositif ait effectué ses tâches ou conservées.

Comme on peut le voir sur la Figure 1, la mémoire 10 reçoit plusieurs types de données distincts qui jouent un rôle dans le fonctionnement du dispositif 2. Ainsi, le moteur 4 accède à des données d'entraînement 12 pour calculer un premier modèle de classification de détection de troubles du rythme cardiaque 14 et un deuxième de détection de troubles du rythme cardiaque 16. Ces données sont ensuite utilisées pour classifier des données d'entrée 18.

Dans l'exemple décrit ici, le moteur 4 est un moteur d'apprentissage automatique (« machine learning » en anglais) supervisé. Ainsi, les données d'entraînement 12 comprennent des électrogrammes cardiaques qui ont été étiquettées (« labeled » en anglais) pour indiquer s'ils sont associés à un trouble cardiaque recherché ou non. Dans l'exemple décrit ici, le trouble cardiaque visé concerne la fibrillation atriale. Dans d'autres modes de réalisation, il pourra s'agir d'un ou plusieurs autres troubles cardiaques.

Toujours en variante, le moteur 4 peut utiliser un autre moteur d'apprentissage automatique, par exemple semi-supervisé ou non supervisé. La flèche en pointillés entre les données d'entrée 18 et le moteur 4 représente la capacité de ce dernier à fonctionner en mode semi-supervisé ou non supervisé. Le moteur 4 pourra également mettre en œuvre une combinaison d'apprentissage automatique supervisé, semi-supervisé et/ou non supervisé. Dans l'exemple décrit ici, le moteur 4 utilise principalement un algorithme de partitionnement de données (« clustering » en anglais).

En variante, le moteur 4 pourrait mettre en œuvre un algorithme de régression, de comparaison (« instance-based » en anglais), de régularisation, un arbre décisionnel, un algorithme bayésien, un réseau de neurones (« neural network » en anglais), un algorithme d'apprentissage profond (« deep learning » en anglais), ou une combinaison de ceux-ci.

En variante, le moteur 4 peut être omis, et le dispositif 2 peut fonctionner sur la base de modèles de détection de troubles du rythme cardiaque prédéfinis.

Dans le mode de réalisation décrit ici, les données d'entrée représentent des électrogrammes cardiaques, c'est-à-dire des signaux électriques tirés de l'activité cardiaque. Pour les traiter, ces électrogrammes sont découpés en séquences d'une durée choisie. De manière préférée, cette durée correspond aux paramètres du modèle de détection de troubles du rythme cardiaque utilisé. Cela signifie que, en général, la durée avec laquelle sont découpées les données d'électrogrammes pour la détermination du modèle pour le moteur 4 est la même que la durée des données d' électrogrammes utilisées par le dispositif 2 lors de son fonctionnement opérationnel.

Ainsi, le pilote 8 reçoit les données d'entrée et les découpe selon la durée associée au modèle avec lequel la classification est souhaitée, puis le classifieur 6 est appelé avec le modèle et les données découpées.

La Demanderesse a découvert que la détection était plus efficace lorsque deux modèles sont utilisés. Pour cela, le premier modèle de détection de troubles du rythme cardiaque est établi en utilisant une première durée pour les données d'électrogrammes, dans l'exemple décrit ici de 5 secondes, tandis que le deuxième modèle de détection de troubles du rythme cardiaque est établi en utilisant une deuxième durée qui est un multiple entier de la première durée. Dans l'exemple décrit ici, le multiple entier est égal à 5. En variante, il pourrait varier entre 2 et 10.

Dans l'exemple décrit ici, ces deux modèles utilisent le plus petit de deux estimateurs calculés à partir des données d'électrogrammes. Ces estimateurs visent à qualifier la durée de cycle associée aux données d'électrogrammes. Pour cela, les données d'électrogrammes sont stockées dans un vecteur dont chaque élément correspond à un échantillon du signal représenté par les données d'électrogrammes. Ensuite, un paramètre d'autocorrélation T est utilisé pour définir deux vecteurs de taille T : un premier vecteur comprenant les T premiers échantillons du vecteur de données d'électrogrammes, et un deuxième vecteur comprenant les T derniers échantillons du vecteur de données d'électrogrammes.

Dans l'exemple décrit ici, le premier estimateur est calculé en mesurant une auto-corrélation normalisée en réalisant le produit scalaire du premier vecteur et du deuxième vecteur, divisé par le produit des normes euclidiennes du premier vecteur et du deuxième vecteur. En faisant varier T, on détermine une valeur maximale et une valeur minimale du premier estimateur, puis la valeur retenue pour le premier estimateur est choisie comme étant celle pour laquelle la valeur de T est la plus petite et telle que le premier estimateur calculé avec cette valeur de T est plus grand que la différence entre la valeur maximale du premier estimateur retranchée de 0,3 fois la différence entre la valeur maximale et la valeur minimale du premier estimateur. En variante, le premier estimateur peut être déterminé différemment, par exemple en changeant le coefficient de 0,3, ou en recherchant une valeur de T optimisant l'estimateur, de manière empirique, exhaustive, ou encore par machine learning.

Dans l'exemple décrit ici, le deuxième estimateur est calculé en calculant pour chaque valeur de T la norme euclidienne au carré de la différence entre le premier vecteur et le deuxième vecteur, divisée par le produit de l'échantillon de valeur absolue la plus importante dans le premier vecteur par l'échantillon de valeur absolue la plus importante dans le deuxième vecteur. Comme pour le premier estimateur, une valeur maximale et une valeur minimale du deuxième estimateur sont déterminées, puis la valeur retenue pour le deuxième estimateur est choisie comme étant celle pour laquelle la valeur de T est la plus petite et telle que le deuxième estimateur calculé avec cette valeur de T est plus grand que la différence entre la valeur minimale du deuxième estimateur ajoutée de 0,2 fois la différence entre la valeur maximale et la valeur minimale du deuxième estimateur. En variante, le premier estimateur peut être déterminé différemment, par exemple en changeant le coefficient de 0,3, ou en recherchant une valeur de T optimisant l'estimateur, de manière empirique, exhaustive, ou encore par machine learning.

Toujours en variante, le premier modèle et le deuxième modèle peuvent être basés sur d'autres caractéristiques, en complément ou en remplacement des premier et deuxième estimateurs.

La combinaison de ces deux modèles est particulièrement avantageuse car le premier modèle est extrêmement rapide à mettre en œuvre car la première durée est courte, tandis que le deuxième modèle est extrêmement précis même si la deuxième durée est beaucoup plus longue.

Pour note, les électrogrammes cardiaques sont obtenus en déplaçant une électrode dans le cœur au niveau de zones d'intérêt. La vitesse de déplacement de l'électrode est donc cruciale dans la détection, mais ne doit pour autant pas être trop lente afin de limiter les risques opératoires. Cette vitesse de déplacement de l'électrode influe nécessairement sur la précision des mesures. En effet, au fur et à mesure que l'électrode est déplacée d'une première zone vers une seconde zone, les mesures recueillies sont de moins en moins associée à la première zone, et de plus en plus associée à la seconde zone.

Dans le cadre de l'invention, cela peut rendre le deuxième modèle de détection de troubles du rythme cardiaque moins efficace du fait de la durée plus importante de la deuxième durée. Ainsi, même si le premier modèle est moins précis, il permet une détection primaire des zones d'intérêt dans lesquelles un praticien doit passer plus de temps afin que le deuxième modèle indique avec certitude si ce sont des zones pertinentes. Il est à noter que cet avantage est aussi bien valable pour une utilisation du dispositif selon l'invention en temps réel pendant une procédure qu'en mode différé, afin de limiter les faux négatifs, c'est-à-dire les zones qui auraient dû être détectées comme pertinentes, mais pour lesquelles le praticien est passé « trop vite ».

De manière classique, lorsque le classifieur 6 applique le premier modèle de détection de troubles du rythme cardiaque ou le deuxième modèle de détection de troubles du rythme cardiaque à des données d'électrogrammes cardiaques, il retourne en sortie une valeur. Cette valeur représente une probabilité que les données d'électrogrammes en question soient considérées comme significatives d'une détection d'un trouble du rythme cardiaque.

Avantageusement, lorsque le pilote 8 applique le premier modèle de détection de troubles du rythme cardiaque à des données d'électrogrammes cardiaques et que la valeur de réponse excède un seuil choisi de détection (par exemple 70%), alors des données d'alerte 20 sont émises. Néanmoins, ce n'est que lorsque le deuxième modèle le premier modèle de détection de troubles du rythme cardiaque retourne une valeur de réponse qui excède un second seuil choisi (par exemple 80%) qu'une zone dont ont été tirée les données d'électrogrammes cardiaques est considérée comme pertinente. Ainsi, les données d'alerte 20 permettent d'indiquer qu'une zone est d'intérêt, et mérite d'être analysée plus en détail afin que le deuxième modèle soit appliqué de manière optimale.

La Figure 2 représente un exemple de mise en œuvre d'une fonction de détection par le pilote 8.

Cette fonction commence par une opération 200 dans laquelle les données d'entrée sont reçues, ainsi que les paramètres d'exécution comme la première durée et la deuxième durée d2, ainsi que les seuils de détection s1 pour le premier modèle et s2 pour le deuxième modèle.

Classiquement, les données d'entrée sont reçues par paquet. La Figure 2 représente le traitement d'un paquet, la fonction se répétant pour chaque nouveau paquet. Pour des raisons de simplicité de présentation, l'exemple décrit ici traite le cas où un paquet de données correspond à la deuxième durée. Dans le cas où les paquets sont plus grands ou plus petits que la deuxième durée, la fonction de la Figure 2 peut être aisément adaptée pour tenir compte des données manquantes ou en trop.

Ensuite, dans une opération 210, les données sont découpées selon la première durée, puis un indice i est initialisé à 0 et le multiple entier correspondant à la division de la deuxième durée par la première durée est calculé dans une opération 220.

Une boucle est alors lancée afin d'appliquer le première modèle avec chaque morceau des données d'entrée ayant la première durée, et le deuxième modèle à l'ensemble.

Pour cela, un test de sortie de boucle vérifie dans une opération si l'indice i est strictement inférieur au multiple k dans une opération 230. Lorsque c'est le cas, le premier modèle est appliqué au bloc des données d'entrées ayant l'indice i dans une opération 240, et la valeur v1 résultante est comparée au seuil s1 dans une opération 250. Si la valeur v1 excède le seuil s1, alors les données d'alerte sont émises dans une opération 255. Dans le cas contraire, ou après l'opération 255, l'indice i est incrémenté dans une opération 260 et la boucle reprend avec le test de l'opération 230. Dans l'exemple décrit ici, la valeur de seuil s1 peut être fixée à 0,5. En variante, elle peut être fixée différemment et/ou faire l'objet d'une optimisation.

Lorsque tous les blocs de données ont été parcourus, une opération 270 applique le deuxième modèle avec l'ensemble des blocs, puis la valeur résultante v2 est comparée au seuil s2 dans une opération 280. Si la valeur v2 excède le seuil s2, alors les données de détection sont émises dans une opération 285. Dans le cas contraire, ou après l'opération 285, la fonction se termine dans une opération 299. Dans l'exemple décrit ici, la valeur de seuil s2 peut être fixée à 0,7. En variante, elle peut être fixée différemment et/ou faire l'objet d'une optimisation.

Il convient de noter que la classification de l'opération 270 peut être réalisée en appliquant un modèle à part entière, ou encore en réalisant des opérations sur la bases des valeurs tirées de l'application du premier modèle. Ainsi, la valeur v2 peut être une combinaison linéaire des valeurs v1 calculées dans la boucle, par exemple avec une pondération d'autant plus importante que les valeurs v1 associées à un indice i élevé. En effet, plus l'indice i est faible, plus les données correspondantes sont éloignées temporellement de l'instant de détection, et plus l'électrode risque d'être éloignée de la zone concernée.

En variante, d'autres types de fonctions peuvent être mises en œuvre, comme une moyenne arithmétique, seuillée, ou toute combinaison basée sur les valeurs v1.

Toujours en variante, un cathéther dit de référence peut être placé au niveau d'une zone du cœur dont on sait qu'elle est saine, et les données d'électrogrammes qui en découlent être traitées pour déterminer le premier estimateur et le deuxième estimateur, comme décrit plus haut, afin d'en tirer la valeur d'estimateur la plus faible. Cette valeur peut être comparée à la valeur déterminée de manière similaire sur les données d'électrogrammes courantes, et une alerte similaire à celle de l'opération 255 être déclenchée si une différence de plus de 150ms est déterminée entre ces valeurs, ou si les données d'électrogrammes courantes donnent une valeur inférieure à 150ms.

## Revendications

1. Dispositif de détection de zones cardiaques favorisant la fibrillation atriale, **caractérisé en ce qu'**il comprend une mémoire (10) agencée pour recevoir des données d'électrogrammes cardiaques et stockant des données définissant un premier modèle de classification de détection de troubles du rythme cardiaque et un second modèle de classification de détection de troubles du rythme cardiaque, le premier modèle de classification de détection de troubles du rythme cardiaque découlant du traitement par apprentissage automatique de données associées à des électrogrammes cardiaques ayant une première durée, et le deuxième modèle de classification de détection de troubles du rythme cardiaque découlant du traitement par apprentissage automatique de données associées à des électrogrammes cardiaques ayant une deuxième durée, la première durée étant inférieure à la deuxième durée, un classifieur (6) agencé pour analyser des données d'électrogrammes cardiaques sur la base d'un modèle de classification, et pour renvoyer une valeur de classification, et un pilote (8) agencé pour stocker dans la mémoire (10) des données d'électrogrammes cardiaques reçues en entrée, d'une part pour regrouper ces données selon la première durée et les analyser avec le classifieur (6) sur la base du premier modèle de classification de détection de troubles du rythme cardiaque et d'autre part pour regrouper ces données selon la deuxième durée et les analyser avec le classifieur (6) sur la base du deuxième modèle de classification de détection de troubles du rythme cardiaque, et pour retourner des données d'alerte lorsque l'analyse par le classifieur (6) sur la base du premier modèle de classification retourne une valeur de classification associée à un trouble du rythme cardiaque.

2. Dispositif selon la revendication 1, dans lequel la deuxième durée est un multiple entier de la première durée, et dans lequel le pilote (8) est agencé pour appliquer le deuxième modèle de classification de détection de troubles du rythme cardiaque de sorte que la valeur de classification pour des données d'électrogrammes ayant la deuxième durée correspond à une combinaison linéaire des valeurs de classification obtenues en découpant les données d'électrogrammes ayant la deuxième durée en une pluralité de sous-groupes de données d'électrogrammes ayant la première durée et en appliquant le premier modèle de classification de détection de troubles du rythme cardiaque à chacun de ces sous-groupes de données d'électrogrammes.

3. Dispositif selon la revendication 2, dans lequel le pilote (8) est agencé pour calculer une moyenne pondérée des valeurs de classification obtenues en appliquant le premier modèle de classification de détection de troubles du rythme cardiaque à chacun des sous-groupes de données d'électrogrammes.

4. Dispositif selon l'une des revendications précédentes, comprenant en outre un moteur d'apprentissage automatique (4) agencé pour déterminer le premier modèle de classification de détection de troubles du rythme cardiaque et le deuxième modèle de classification de détection de troubles du rythme cardiaque.

5. Dispositif selon la revendication 4, dans lequel le moteur d'apprentissage (4) est agencé pour exécuter un apprentissage choisi parmi un apprentissage supervisé, un apprentissage semi-supervisé, un apprentissage non-supervisé, ou une combinaison de deux ou plus de ces apprentissages.

## Patentansprüche

1. Vorrichtung zur Erkennung von Herzbereichen, die Vorhofflimmern begünstigen, **dadurch gekennzeichnet, dass** sie einen Speicher (10) umfasst, der so beschaffen ist, dass er Daten von Herzelektrogrammen empfängt und Daten speichert, die ein erstes Klassifizierungsmodell für die Erkennung von Herzrhythmusstörungen und ein zweites Klassifizierungsmodell für die Erkennung von Herzrhythmusstörungen definieren, wobei das erste Klassifizierungsmodell zur Erkennung von Herzrhythmusstörungen aus der Verarbeitung durch maschinelles Lernen von Daten abgeleitet wird, die mit Herzelektrogrammen mit einer ersten Dauer verknüpft sind, und das zweite Klassifizierungsmodell zur Erkennung von Herzrhythmusstörungen aus der Verarbeitung durch maschinelles Lernen von Daten abgeleitet wird, die mit Herzelektrogrammen mit einer zweiten Dauer verknüpft sind, wobei die erste Dauer kleiner als die zweite Dauer ist, einen Klassifizierer (6), der so beschaffen ist, dass er Herzelektrogrammdaten basierend auf einem Klassifizierungsmodell analysiert und einen Klassifizierungswert zurückgibt, und einen Treiber (8), der so beschaffen ist, dass er die als Eingabe empfangenen Herzelektrogrammdaten im Speicher (10) speichert, um einerseits diese Daten nach der ersten Dauer zu gruppieren und sie mit dem Klassifizierer (6) basierend auf dem ersten Klassifizierungsmodell zur Erkennung von Herzrhythmusstörungen zu analysieren und um andererseits diese Daten nach der zweiten Dauer zu gruppieren und sie mit dem Klassifizierer (6) basierend auf dem zweiten Klassifizierungsmodell zur Erkennung von Herzrhythmusstörungen zu analysieren und Warndaten zurückzugeben, wenn die Analyse durch den Klassifizierer (6) basierend auf dem ersten Klassifizierungsmodell einen mit einer Herzrhythmusstörung verknüpften Klassifizierungswert zurückgibt.

2. Vorrichtung nach Anspruch 1, wobei die zweite Dauer ein ganzzahliges Vielfaches der ersten Dauer ist, und wobei der Treiber (8) so beschaffen ist, dass er das zweite Klassifizierungsmodell zur Erkennung von Herzrhythmusstörungen anwendet, so dass der Klassifizierungswert für Elektrogrammdaten mit der zweiten Dauer einer linearen Verknüpfung der Klassifizierungswerte entspricht, die durch Aufteilen der Elektrogrammdaten mit der zweiten Dauer in eine Vielzahl von Untergruppen von Elektrogrammdaten mit der ersten Dauer und durch Anwenden des ersten Klassifizierungsmodells zur Erkennung von Herzrhythmusstörungen auf jede dieser Untergruppen von Elektrogrammdaten erhalten werden.

3. Vorrichtung nach Anspruch 2, wobei der Treiber (8) so beschaffen ist, dass er einen gewichteten Mittelwert der Klassifizierungswerte berechnet, die durch Anwenden des ersten Klassifizierungsmodells zur Erkennung von Herzrhythmusstörungen auf jede der Untergruppen von Elektrogrammdaten erhalten werden.

4. Vorrichtung nach einem der vorhergehenden Ansprüche, ferner umfassend eine maschinelle Lern-Engine (4), die so beschaffen ist, dass sie das erste Klassifizierungsmodell zur Erkennung von Herzrhythmusstörungen und das zweite Klassifizierungsmodell zur Erkennung von Herzrhythmusstörungen bestimmt.

5. Vorrichtung nach Anspruch 4, wobei die Lern-Engine (4) so beschaffen ist, dass sie ein Lernen ausführt, das aus einem überwachten Lernen, einem halbüberwachten Lernen, einem nicht überwachten Lernen oder einer Kombination aus zwei oder mehr dieser Lernvorgänge ausgewählt ist.

## Claims

1. A device for detecting areas of heart conducive to atrial fibrillation, **characterised in that** it comprises a memory (10) arranged to receive cardiac electrogram data and storing data defining a first classification model for detecting heart rhythm disorders and a second classification model for detecting heart rhythm disorders, the first classification model for detecting heart rhythm disorders resulting from machine learning processing of data associated with cardiac electrograms having a first duration, and the second classification model for detecting heart rhythm disorders resulting from machine learning processing of data associated with cardiac electrograms having a second duration, the first duration being less than the second duration, a classifier (6) arranged to analyse cardiac electrogram data on the basis of a classification model, and to return a classification value, and a driver (8) arranged to store in the memory (10) cardiac electrogram data received as input, on the one hand to aggregate these data according to the first duration and analyse them with the classifier (6) on the basis of the first classification model for detecting heart rhythm disorders and, on the other hand to aggregate these data according to the second duration and analyse them with the classifier (6) on the basis of the second classification model for detecting heart rhythm disorders, and to return alert data when the analysis by the classifier (6) on the basis of the first classification model returns a classification value associated with a heart rhythm disorder.

2. The device according to claim 1, wherein the second duration is an integer multiple of the first duration, and wherein the driver (8) is arranged to apply the second classification model for detecting heart rhythm disorders such that the classification value for electrogram data having the second duration corresponds to a linear combination of the classification values obtained by cutting the electrogram data having the second duration into a plurality of sub-groups of electrogram data having the first duration and by applying the first classification model for detecting heart rhythm disorders to each of these sub-groups of electrogram data.

3. The device according to claim 2, wherein the driver (8) is arranged to compute a weighted average of the classification values obtained by applying the first classification model for detecting heart rhythm disorders to each of the sub-groups of electrogram data.

4. The device according to one of the preceding claims, further comprising a machine learning engine (4) arranged to determine the first classification model for detecting heart rhythm disorders and the second classification model for detecting heart rhythm disorders.

5. The device according to claim 4, wherein the learning engine (4) is arranged to carry out learning selected from among supervised learning, semi-supervised learning, unsupervised learning, or a combination of two or more of these learnings.
